# EUROPEAN PATENT APPLICATION

(11) **EP 2 351 795 A1**
(43) Date of publication of application: **03.08.2011**
(21) Application number: 09816006.2
(22) Date of filing: 14.08.2009
(51) Int. Cl.: C09B 61/00, C07D 471/14

(54) **DYE COMPOUND, PROCESS FOR PRODUCING SAME, AND COLORING AGENT**

(30) Priority: 25.09.2008 JP 2008245585
(71) Applicant: Incorporated Administrative Agency National Agriculture and Food Research Organization, Ibaraki 305-8517 (JP); KNC Laboratories Co., Ltd, Kobe-shi, Hyogo 651-2271 (JP)
(72) Inventor: NAKANO Hiroshi, Chikugo-shi Fukuoka 833-0041 (JP); SAKAI Makoto, Chikugo-shi Fukuoka 833-0041 (JP); KAJI Ryota, Yotsuya, Daisen-shi Akita 014-0102 (JP); YOSHIDA Mitsuru, Tsukuba-shi Ibaraki 305-8642 (JP); KOSEMURA Seiji, Yokohama-shi Kanagawa 223-8521 (JP); SUZUKI Toshisada, Kida-gun Kagawa 761-0795 (JP); HIROSE Katsutoshi, Kobe-shi Hyogo 651-2271 (JP)
(74) Representative: Vossius & Partner
(86) International application number: PCT/JP2009/064353
(87) International publication number: WO 2010/035589

(57) **Abstract**

An object of the present invention is to provide a novel plant-derived pigment compound, which is a pigment compound represented by Formula II: 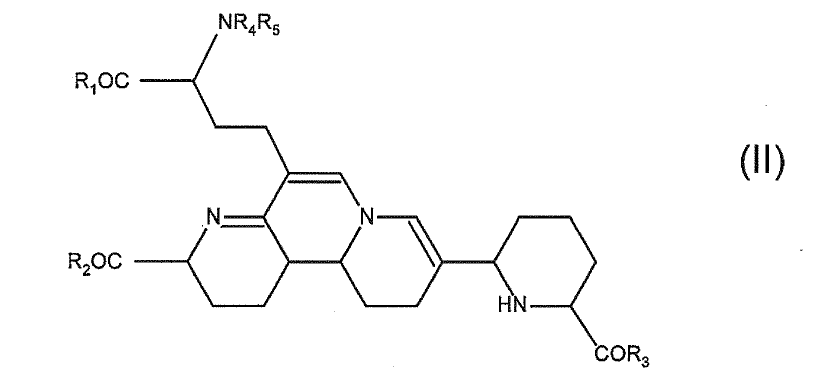 (wherein R₁ to R₃ independently represent a hydroxyl group or an alkoxy group having 1 to 4 carbon atoms, and R₄ and R₅ independently represent a hydrogen atom, an acyl group having 1 to 4 carbon atoms, or an alkyl group having 1 to 4 carbon atoms). The compound is a yellow pigment extracted from the endosperm or the like of the rice cultivar "Hatsuyamabuki."

## Description

### Technical Field

The present invention relates to a pigment compound, a method of producing the same, and a coloring agent. More specifically, the present invention relates to a pigment compound preferably used as a naturally derived coloring agent, a method of producing the same, and a coloring agent. In addition, the "pigment compound" of the present invention includes not only a compound that takes on a yellow color etc. but also a colorless analogous compound thereof.

### Background Art

Hitherto, coloring agents having different color tones have been earnestly awaited in a variety of industrial fields. In particular, coloring agents containing naturally derived pigments obtained from plant extractives and the like can be widely used in technical fields related to foods, pharmaceutical products, cosmetics, industrial chemicals, and so on.

Known examples of naturally derived edible yellow pigments are carotenoid substances (crocin and crocetin of gardenia, safflomin of oil palm (*Elaeis guineensis*), and lutein of marigolds) and flavonoid substances (curcumin of turmeric) (Non-Patent Document 1).

In addition, Patent Document 1 discloses a method of purifying a gardenia yellow pigment by treating a gardenia extract with a chelate resin without adsorptive resin treatment or membrane separation treatment. Further, Patent Document 2 discloses a method of producing a safflower yellow pigment that comprises steps of: allowing a safflower extract to come into contact with activated carbon for adsorption of solid matter containing a pigment component and extracting the pigment component with an alkaline solution.

### Prior Art Documents

### Patent Documents

Patent Document 1: JP Patent Publication (Kokai) No. 2004-131633 A
Patent Document 2: JP Patent Publication (Kokai) No. 10-306224 A (1998)

### Non-Patent Documents

Non-Patent Document 1: Eiyogaku/Shokuhingaku Kyoiku Kenkyukai (Nutritiology/Sitology Educational Working Group) (ed), "ESKKA Food Chemistry," DOBUNSHOIN, pp.107 to 121

### Disclosure of the Invention

### Problem to be Solved by the Invention

Known naturally derived yellow pigments used for foods and the like are carotenoid and flavonoid substances. The color tones thereof are limited. Therefore, new development of an excellent naturally derived yellow pigment has been awaited.

The Rice Breeding Unit of the National Agricultural Research Center for the Kyushu Okinawa Region nurtured the cultivar "Hatsuyamabuki" (previous lineage name: Saikaioh 256), which has a feature of taking on a yellow color throughout its entire endosperm and was the first such case reported, having no precident, by mutating the rice cultivar "Kinuhikari." However, the main component of the yellow substance of "Hatsuyamabuki" still has not been identified.

In view of the above, an object of the present invention is to provide a novel plant-derived pigment compound by identifying the yellow substance of "Hatsuyamabuki" (rice cultivar). In addition, another object of the present invention is to provide a coloring agent comprising the pigment compound as a component.

### Means for Solving Problem

In order to achieve the above objects, the present inventors have succeeded in isolating a yellow substance and a colorless substance from an alcohol/water extractive of the endosperm of "Hatsuyamabuki." The present inventors revealed that the substance is an unknown novel substance that differs from conventional carotenoid or flavonoid pigments by analyzing physicochemical properties of the yellow substance and the colorless substance. In addition, the present inventors established an industrial production method thereof. This has led to the completion of the present invention. Specifically, the present invention is summarized as follows.
(1) A pigment compound of Formula I: 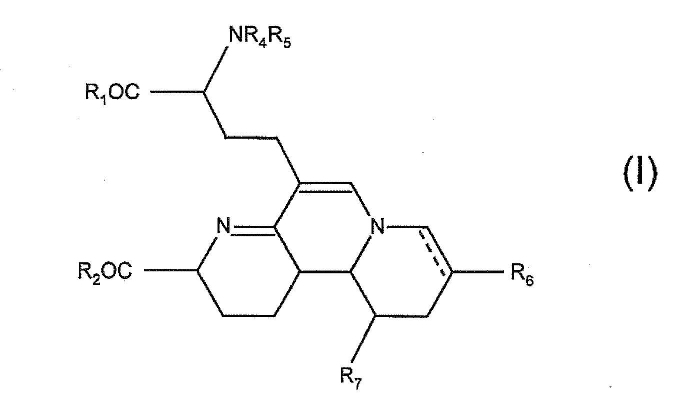 [wherein R₁ and R₂ independently represent a hydroxyl group or an alkoxy group having 1 to 4 carbon atoms, R₄ and R₅ independently represent a hydrogen atom, an acyl group having 1 to 4 carbon atoms, or an alkyl group having 1 to 4 carbon atoms, the following combination of solid and dashed lines represents a single bond or double bond, and R₆ and R₇ independently represent a hydrogen atom or a linear, branched, or cyclic alkyl group or nitrogen-containing heterocyclic group that may have an amino group and/or -COR₈ (wherein R₈ represents a hydroxyl group or an alkoxy group having 1 to 4 carbon atoms)]: 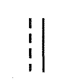
(2) The pigment compound according to (1), wherein the compound of Formula I is a compound of Formula II: 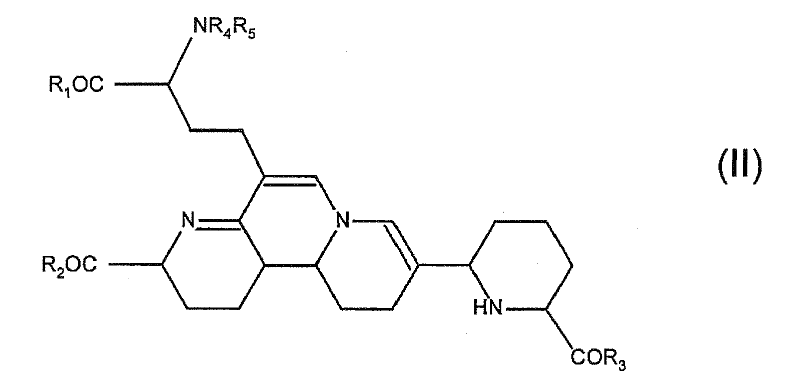 [wherein R₁ to R₃ independently represent a hydroxyl group or an alkoxy group having 1 to 4 carbon atoms, and R₄ and R₅ independently represent a hydrogen atom, an acyl group having 1 to 4 carbon atoms, or an alkyl group having 1 to 4 carbon atoms].
(3) The pigment compound according to (2), wherein the compound of Formula II is a compound of Formula III: 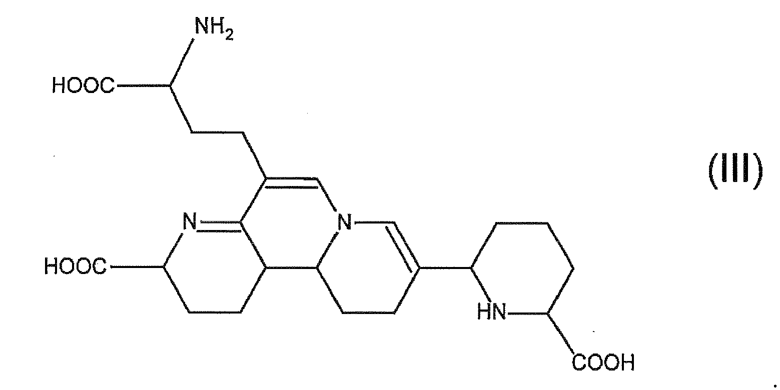
(4) The pigment compound according to (1), wherein the compound of Formula I is a compound of Formula IV: 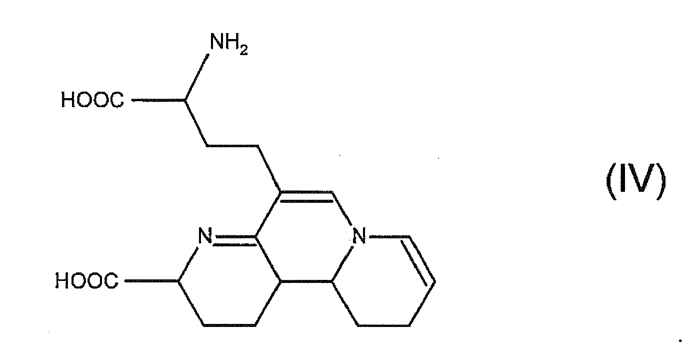
(5) The pigment compound according to (1), wherein the compound of Formula I is a compound of Formula V: 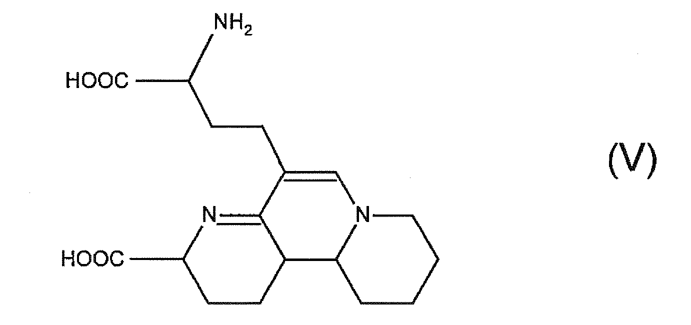
(6) The pigment compound according to (1), wherein the compound of Formula I is a compound of Formula VI: 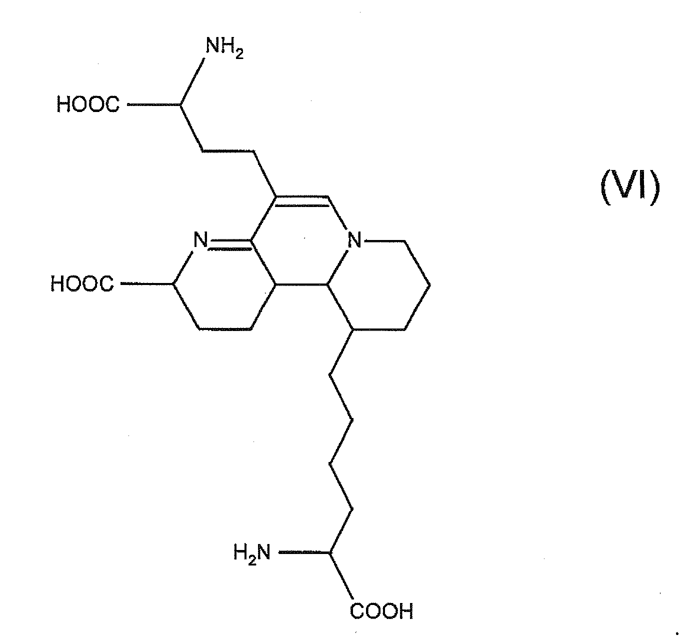
(7) A pigment compound of Formula VII: 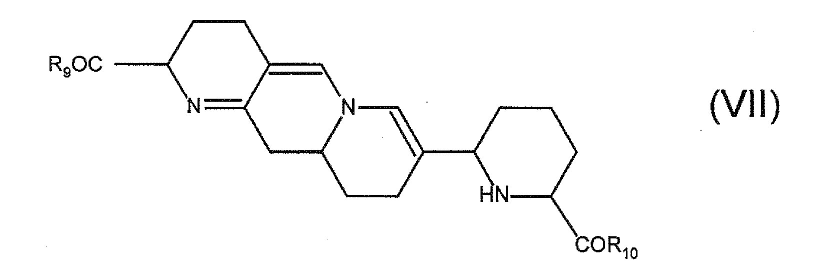 [wherein R₉ and R₁₀ independently represent a hydroxyl group or an alkoxy group having 1 to 4 carbon atoms].
(8) The pigment compound according to (7), wherein the compound of Formula VII is a compound of Formula VIII: 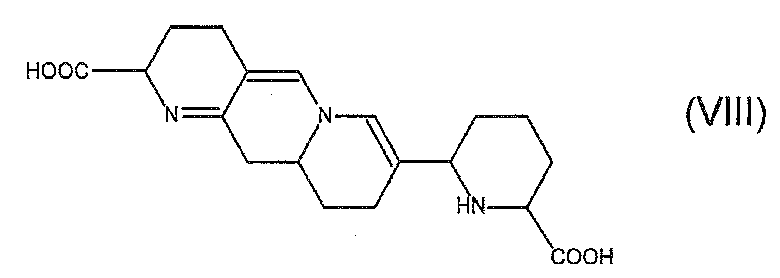
(9) A method of producing the pigment compound according to any one of (3) to (6) or (8), which is a method of producing a pigment compound comprising subjecting seeds, husked rice, endosperm, or bran of the rice cultivar "Hatsuyamabuki" (FERM BP-11149) or its progeny to extraction with water or an alcohol aqueous solution and carrying out separation/purification of the pigment compound from the extract.
(10) A coloring agent comprising an extractive obtained by subjecting seeds, husked rice, endosperm, or bran of the rice cultivar "Hatsuyamabuki" (FERM BP-11149) or its progeny to extraction with water or an alcohol aqueous solution and removing starch from the resultant.
(11) The coloring agent according to (10), which comprises a fraction obtained by allowing the extractive from which starch has been removed to further adsorb to a stationary phase and then to be eluted with a solvent.
(12) A coloring agent comprising the pigment compound according to any one of (1) to (8).

This description includes part or all of the contents as disclosed in the description and/or drawings of Japanese Patent Application No. 2008-245585, which is a priority document of the present application.

### Effects of the Invention

A novel yellow or colorless pigment compound obtained by the present invention can be effectively used as a plant-derived coloring agent in technical fields related to foods, pharmaceutical products, cosmetics, industrial chemicals, and so on.

### Brief Description of the Drawings

Fig. 1 shows the structure of the pigment compound of the present invention.
Fig. 2 shows the ultraviolet absorption spectrum of the pigment compound of the present invention.

### Best Mode for Carrying Out the Invention

The present invention is described below in detail.

The pigment compound of the present invention is represented by Formula I:

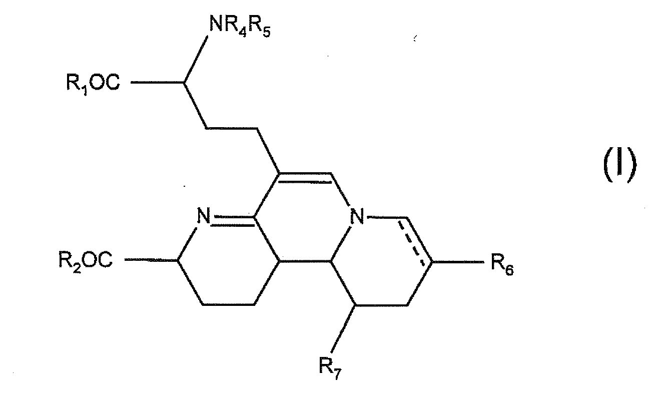

In Formula I, R₁ and R₂ independently represent a hydroxyl group or an alkoxy group having 1 to 4 carbon atoms, R₄ and R₅ independently represent a hydrogen atom, an acyl group having 1 to 4 carbon atoms, or an alkyl group having 1 to 4 carbon atoms, the following combination of solid and dashed lines represents a single bond or double bond, and R₆ and R₇ independently represent a hydrogen atom or a linear, branched, or cyclic alkyl group or nitrogen-containing heterocyclic group that may have an amino group and/or -COR₈ (wherein R₈ represents a hydroxyl group or an alkoxy group having 1 to 4 carbon atoms):

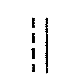

Regarding R₁, R₂ and R₈, examples of an alkoxy group having 1 to 4 carbon atoms include a methoxy group, an ethoxy group, a propoxy group, and a butoxy group. In addition, examples of an acyl group having 1 to 4 carbon atoms include an acetyl group, a propionyl group, and a butyryl group. Further, examples of an alkyl group having 1 to 4 carbon atoms include linear or branched alkyl groups such as a methyl group, an ethyl group, an n-propyl group, an isopropyl group, an n-butyl group, an isobutyl group, a sec-butyl group, and a tert-butyl group.

In addition, an example of "a linear, branched, or cyclic alkyl group or nitrogen-containing heterocyclic group that may have an amino group and/or -COR₈" is a linear, branched, or cyclic alkyl group or nitrogen-containing heterocyclic group having the structure represented by, for example, "-(CH₂)ₙCH(COOH)(NH₂)." In this case, an alkyl group and a heterocyclic group each have preferably 1 to 8 and particularly preferably 4 to 6 carbon atoms (excluding the carbon atoms of "-COR₈"). In addition, when a ring is formed, the ring may contain a nitrogen atom of an amino group as a ring member and thus have a secondary amino group (-NH-).

Preferably, the pigment compound of the present invention has the structure of Formula II:

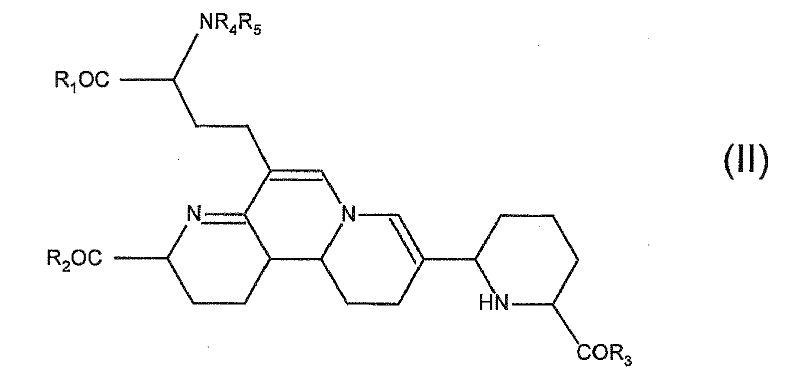

In Formula II, R₁, R₂, R₄, and R₅ are defined as above. R₃ represents a hydroxyl group or an alkoxy group having 1 to 4 carbon atoms. Examples of an alkoxy group having 1 to 4 carbon atoms include a methoxy group, an ethoxy group, a propoxy group, and a butoxy group.

Particularly preferably, the pigment compound of the present invention has the structure of Formula III:

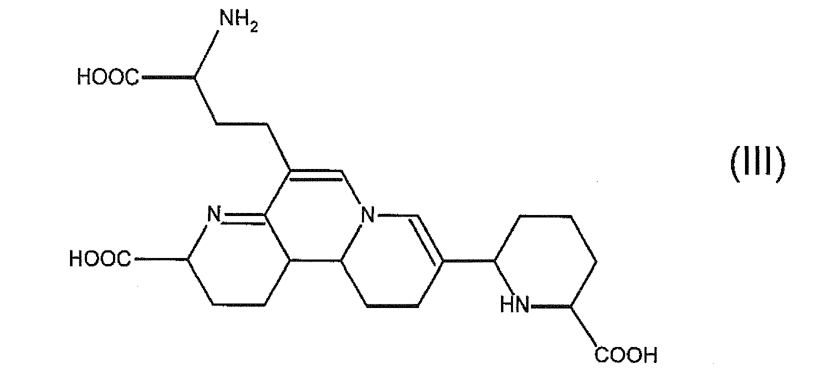

In addition, particularly preferably, the pigment compound of the present invention has the structure of Formula IV:

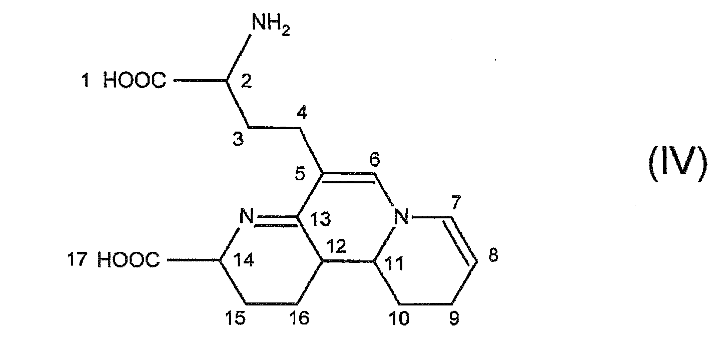

Further, particularly preferably, the pigment compound of the present invention has the structure of Formula V:

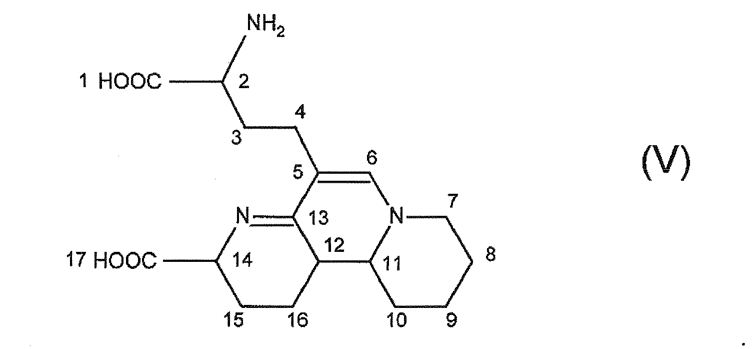

Furthermore, particularly preferably, the pigment compound of the present invention has the structure of Formula VI:

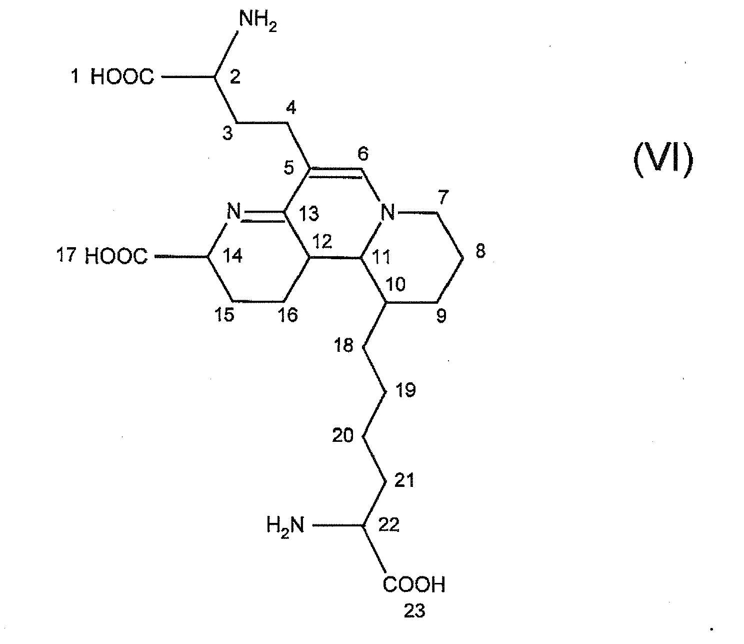

In another embodiment, the pigment compound of the present invention is represented by Formula VII:

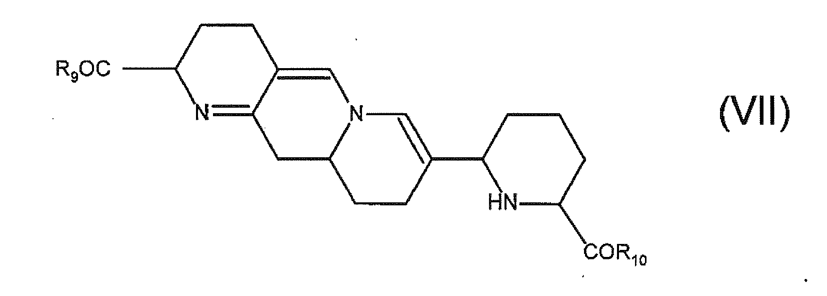

In Formula I, R₉ and R₁₀ independently represent a hydroxyl group or an alkoxy group having 1 to 4 carbon atoms. Examples of an alkoxy group having 1 to 4 carbon atoms include a methoxy group, an ethoxy group, a propoxy group, and a butoxy group.

Preferably, the pigment compound of the present invention has the structure of Formula VIII:

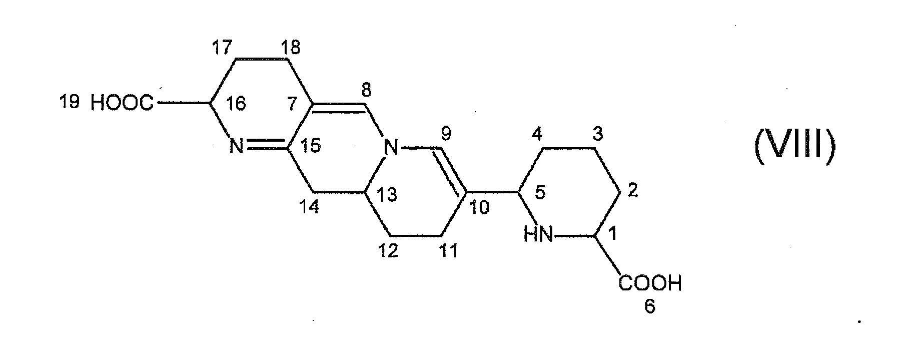

The pigment compounds of Formulae III to VI and VIII can be obtained from seeds (seeds with husk), husked rice, endosperm (polished rice), or bran of the rice cultivar "Hatsuyamabuki" (previous lineage name: Saikaioh 256) or its progeny. "Hatsuyamabuki" was nurtured from seeds of "Kinuhikari" subjected to γ-ray irradiation (irradiation dose: 300 Gy; dose rate: 15 Gy/h) for the purpose of growing a cultivar having novel characteristics at the Institute of Radiation Breeding, the National Institute of Agrobiological Sciences (NIAS), in May 1998. Thereafter, the M₁ generation was cultivated in seedling trays in an uncontrolled outdoor environment at the Tropical Agriculture Research Front, the Japan International Research Center for Agricultural Sciences, in June 1998. Then, the M₂ generation was cultivated in 1999 and the M₃ seeds were collected from each line in the field of the Kyushu Agricultural Experiment Station (currently named "the National Agricultural Research Center for Kyushu Okinawa Region"). The mutants of M₃ generation whose husked rice took on a yellow color were selected by appearance observation, followed by selection for fixation of characteristics by a pedigree breeding.

In 2002, the M₅ generation of the selected mutant was subjected to a performance test and a characteristic certification test under the name "Izumi 1275." Accordingly, the mutant was found to be a yellow endosperm mutant taking on a yellow color even in the form of polished rice. Also after 2003, the mutant was subjected to a performance test and a characteristic certification test. Accordingly, fixation of major characteristics such as yellow endosperm was achieved in practice. From 2005, the M₈ generation was given the local name "Saikaioh 256." In November 2008, the appliation of the cultivar was submitted for registration with the cultivar name "Hatsuyamabuki" in accordance with the Plant Variety Protection and Seed Act. The application was disclosed in February 2009 (Application No.: 23176). Properties of "Hatsuyamabuki" are described below.

### 1) Morphological characteristics

When compared with "Kinuhikari," "Hatsuyamabuki" has a relatively shorter culm length, a similar ear length, and fewer ears, and thus it can be classified as the intermediate type rice. It is an awnless cultivar characterized by electing flag leaf, favorable standing statue and ripening color, moderate grain density of panicle, yellowish white apiculus color and hard shattering habit of grain.

### 2) Ecological characteristics

The panicle heading stage of "Hatsuyamabuki" is similar to that of "Kinuhikari," and it starts 5 days earlier than that for "Mineasahi" The maturing stage thereof is similar to that of "Kinuhikari" and it starts about 2 days earlier than that for "Mineasahi." "Hatsuyamabuki" is an *uruchi* rice (conventional rice) variety classified as a very early variety in a warm place. Regarding yielding ability, "Hatsuyamabuki" is slightly inferior to "Kinuhikari." Regarding lodging resistance, "Hatsuyamabuki" is comparable to "Kinuhikari." Regarding resistance to rice leaf blast and resistance to rice ear blast, "Hatsuyamabuki" is comparable to "Kinuhikari" (a relatively weak level). It is unknown whether or not "Hatsuyamabuki" has resistance to rice stripe disease. Regarding resistance to bacterial leaf blight, "Hatsuyamabuki" is comparable to "Kinuhikari" (a relatively weak level) Regarding viviparity, "Hatsuyamabuki" is comparable to "Kinuhikari" (a relatively easy level).

### 3) Quality and eating quality properties

The thousand-kernel weight of "Hatsuyamabuki" husked rice is approximately 22 g, which is comparable to that of "Kinuhikari." In other words, "Hatsuyamabuki" grains are relatively small (medium-size grains). Regarding quality in terms of the appearance of husked rice, the grain color is relatively yellow. However, the occurrence of white belly, white core, and milk-white grain in "Hatsuyamabuki" can be observed to an extent similar to that in "Kinuhikari." "Hatsuyamabuki" polished rice and boild rice take on a yellow color. The eating quality of "Hatsuyamabuki" is comparable to that of "Nipponbare."

In addition, seeds of "Hatsuyamabuki" were deposited with the International Patent Organism Depositary, National Institute of Advanced Industrial Science and Technology (AIST) (postal code: 305-8566; Tsukuba Central 6, 1-1-1 Higashi, Tsukuba, Ibaraki, Japan) with accession no. FERM P-21664 as of September 3, 2008, and then they were internationally deposited with accession no. FERM BP-11149 as of July 27, 2009 under the Budapest treaty.

In addition, the "progeny" cultivar or lineage of the present invention includes a cultivar or lineage from a hybrid progeny obtained via artificial hybridization using "Hatsuyamabuki" as a female or a male parent. Alternatively, it includes a cultivar or lineage from a progeny obtained by treating seeds or tissues of "Hatsuyamabuki" to cause a mutation or a genetic variation such as a transformation therein. Such a progeny or lienage has the characteristic of having yellow endosperm specific to "Hatsuyamabuki."

A pigment compound can be obtained via solvent extraction from seeds (husked seeds), husked rice, endosperm (polished rice), or bran of "Hatsuyamabuki" or its progeny, followed by separation/purification of the extract. As an extraction solvent, water or an organic solvent is generally used, and water or an alcohol aqueous solution is preferably used. Examples of alcohol include, but are not limited to, methanol, ethanol, and propanol. In some cases, a chelate agent, acid/alkali, or the like can be added as an extractant. If necessary, starch is removed from the obtained extract by means of alcohol precipitation, ultrafiltration, or the like, followed by filtration/concentration and purification. Thus, a pigment compound of interest can be efficiently obtained.

For purification, conventionally known methods can be used. For instance, silica gel chromatography, reversed-phase silica gel chromatography, high-performance liquid chromatography, and the like can be used in combination in an appropriate manner.

The pigment compounds of Formulae III to VI and VIII described above can be obtained by separation/purification from extracts. At such time, a desired compound can be isolated by, for example, changing the composition of an eluent. In addition, derivatives of the compounds of Formulae III to VI and VIII can be obtained by treating the compounds with appropriate reagents after purification or before purification. Specifically, a derivative of the compound of Formula I, Formula II, or Formula VII in which R₁ to R₃, R₉, and R₁₀ are alkoxy groups can be obtained by esterification, i.e. by reacting the relevant compound of Formula III, Formula VIII, or the like with an appropriate alcohol with the addition of a dehydrating agent according to need. In a case in which, in Formula I, R₆ and R₇ independently have -COR₈, a derivative can be obtained via esterification of a carboxyl group in the same manner described above. In addition, an amide derivative in which R₄ and R₅ independently represent an acyl group having 1 to 4 carbon atoms can be obtained by reacting the compound of Formula III or the like with a relevant carboxylic halide or carboxylic anhydride or with carboxylic acid with the use of a dehydrating agent such as DCC. Further, a derivative in which R₄ and R₅ independently represent an alkyl group can be obtained by forming a metal salt of the compound of Formula III or the like in which R₄ and R₅ independently represent a hydrogen atom and treating the metal salt with alkyl halide or by treating it with an alkylated metal reagent comprising copper, bismuth, or the like for N-alkylation. However, the present invention is not limited to such method. In particular, when the pigment compound is used as a coloring agent for foods, pharmaceutical products, cosmetics, and so on, the type of a reagent should be considered in terms of toxicity and other factors.

A compound obtained by the present invention is a novel yellow or colorless pigment compound. Since it is a plant-derived compound, it can be preferably used as a food coloring agent. Also, it can be used as a coloring agent for pharmaceutical products, cosmetics, industrial chemicals, and so on. In addition, pigment compounds of Formulae I to VII can be ionized. For instance, in such case, a carboxyl group is present in the form of COO- in water or the like. Needless to say, the present invention encompasses such ionized pigment compounds.

In addition, when the compound of the present invention is used as a coloring agent, the compound is not necessarily isolated as a pigment compound. A composition containing, as a component, a pigment compound obtained during the step of separation/purification can be used as a coloring agent. Specifically, for instance, seeds, husked rice, endosperm, or bran of the rice cultivar "Hatsuyamabuki" or its progeny are subjected to extraction with water or an alcohol aqueous solution, starch is removed therefrom, and the resultant is dried according to need. The thus obtained extractive (including a mixture of the compounds of Formulae III to VI and VIII) can be directly used as a coloring agent. Herein, the content of the mixture of the compounds of Formulae III to VI and VIII in solid matter of the extractive differs depending on extraction methods. However, in general, the content is 0.01% to 0.50% by weight. Alternatively, an extractive from which starch has been removed is allowed to further adsorb to a stationary phase for column chromatography or the like such that a fraction (usually containing at least one of the compounds of Formulae III to VI and VIII) eluted in an appropriate solvent can be directly used as a coloring agent. In addition, since "Hatsuyamabuki" seeds contain substantially no flavonoid compounds, the obtained coloring agents also contain substantially no flavonoid compounds.

### Examples

The present invention is hereafter described in greater detail with reference to the following examples, although the present invention is not limited thereto.

### (Example 1)

The endosperm of the rice cultivar "Hatsuyamabuki" (previous lineage name: "Saikaioh 256") (20 kg) was subjected to extraction using a methanol aqueous solution (100 L) (methanol : water = 10:90 (v/v)) at 25°C for a day. The extract was filtrated and concentrated at 35°C under reduced pressure, followed by methanol precipitation (3500 x g, 10 minutes, 25°C) with the use of a methanol aqueous solution (methanol : water = 5:1 (v/v)).

Subsequently, the supernatant fraction (184 g) taking on a yellow color was concentrated at 35°C under reduced pressure and fractionated into 6 fractions by Sep-pak C18 cartridge chromatography with the use of methanol aqueous solutions (methanol : water = 0:100, 5:95, 10:90, 15:85, 20:80, and 100:0 (v/v)). The methanol aqueous solution (methanol : water = 10:90 (v/v)) fraction taking on a yellow color (2 g) was concentrated at 35°C under reduced pressure and subjected to high-performance liquid chromatography (ODS-80Ts; 4.6 x 250 mm; TOSOH; methanol : water = 10:90 (v/v); 0.8 ml/minute). A desired pigment compound taking on a yellow color (35 mg) was obtained from the fraction that had taken on a yellow color (retention time: 9 to 11 minutes).

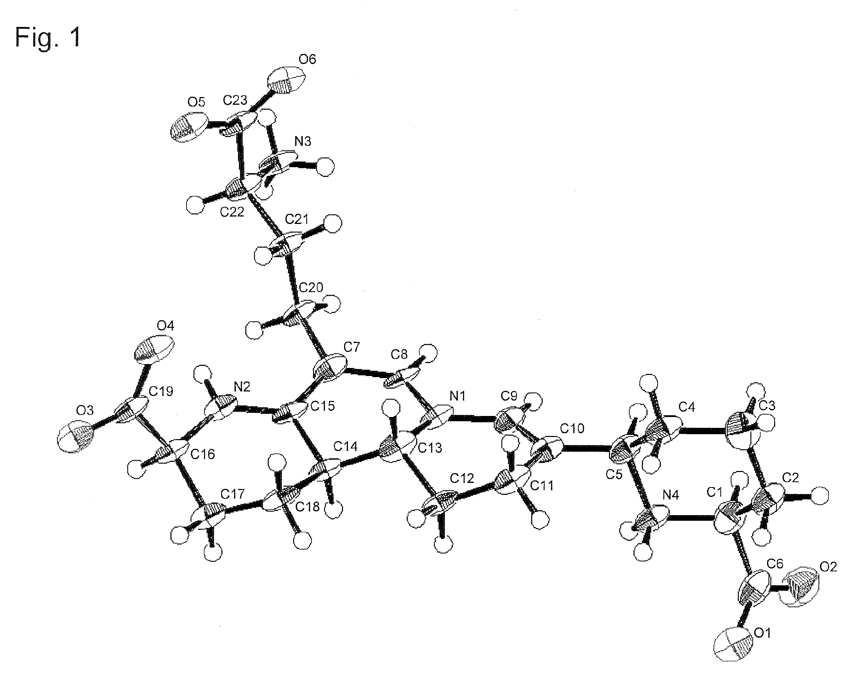

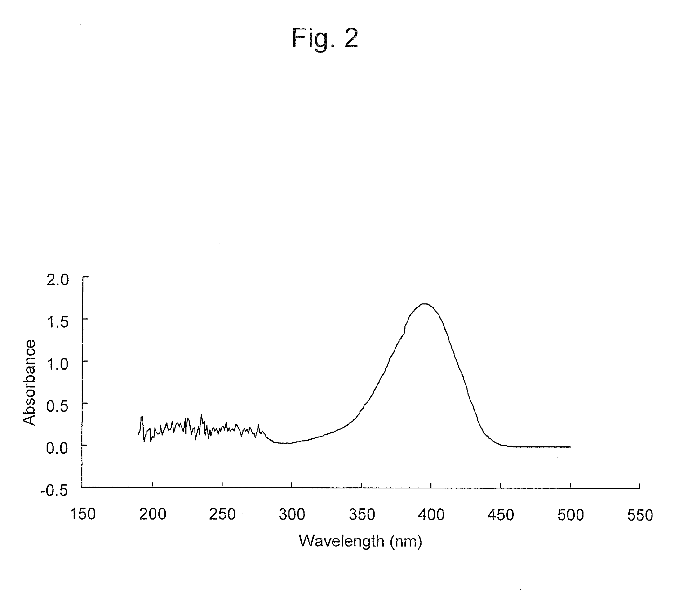

The obtained pigment compound was subjected to single-crystal X-ray structural analysis, high-resolution electrospray ionization mass spectroscopy (HRESIMS), and NMR for structural determination. The following are the apparatuses used for single-crystal X-ray structural analysis: beam line: SPring-8 BL26B1; detector: an Rigaku RAXIS V imaging plate area detector. As a result, the chemical structural formula (Formula III) in a plane view and the absolute structural formula shown in fig. 1 were obtained. In addition, the ultraviolet-visible absorption spectrum (fig. 2) of the compound differed from ultraviolet-visible absorption spectra of carotenoid substances and flavonoid substances. Therefore, the color tone of the compound differed from the color tones of such substances.

Physicochemical properties of the pigment compound are described below. In addition, table 1 gives the ¹³C NMR and ¹H NMR spectral data for the pigment compound (measurement solvent: D₂O).
(1) Appearance:
Yellow powder

(2) Ultraviolet-visible absorption spectrum λₘₐₓ (water):
395 nm (ε17200)

(3) Molecular formula:

C₂₃H₃₂N₄O₆

(4) Molecular weight determined by HRESIMS:
461.247558 (M+H)⁺
459.208096 (M+H)⁻

**[Table 1]**

| No. | δ_{C}^{a} | δ_{H}^{b} | HMBC (H→C) |
|---|---|---|---|
| 1 | 61.5 | 3.47-3.53 | 6 |
| 2 | 26.9 | 2.08-2.13 | |
| | | 1.41-1.50 | |
| 3 | 23.6 | 1.84-1.88 | |
| | | 1.47-1.54 | |
| 4 | 27.4 | 1.73-1.81 | |
| | | 1.55-1.63 | |
| 5 | 61.0 | 3.56-3.63 | 4, 9, 10 |
| 6 | 175.1 | | |
| 7 | 101.8 | | |
| 8 | 153.8 | 7.29 | 7, 9, 13, 15, 20 |
| 9 | 130.7 | 6.52 | 5, 8, 10, 11, 13 |
| 10 | 118.6 | | |
| 11 | 21.7 | 2.11-2.19 | |
| 12 | 26.0 | 2.25-2.32 | |
| | | 1.52-1.58 | |
| 13 | 57.2 | 3.47-3.55 | |
| 14 | 39.9 | 2.78-2.86 | 12, 13, 15, 18 |
| 15 | 167.7 | | |
| 16 | 57.6 | 4.11 | 15, 17, 18, 19 |
| 17 | 25.5 | 2.14-2.22 | |
| | | 1.93-2.02 | |
| 18 | 19.4 | 1.87-1.94 | |
| | | 1.17-1.26 | |
| 19 | 178.0 | | |
| 20 | 23.6 | 2.20-2.28 | 7, 8, 15, 21, 22 |
| 21 | 55.0 | 3.35-3.61 | 20,22,23 |
| 22 | 30.4 | 1.85-1.93 | 7, 20, 21, 23 |
| | | 1.73-1.82 | 7,20,21,23 |
| 23 | 175.3 | | |

| | | | |
|---|---|---|---|
| Measurement at 100 MHz ^{b} Measurement at 400 MHz Each proton multiplicity cannot be read correctly due to overlapping of many protons. | | | |

### (Example 2)

The methanol aqueous solution (methanol : water = 20:80) fraction obtained in Example 1 was concentrated at 35°C under reduced pressure and subjected to high-performance liquid chromatography (ODS-80Ts; 4.6 x 250 mm; TOSOH; methanol : water = 3:17 (v/v); 0.8 ml/minute). A desired pigment compound taking on a yellow color (1.7 mg) was obtained from the fraction taking on a yellow color (retention time: 10 to 15 minutes).

The obtained pigment compound was subjected to single-crystal X-ray structural analysis, high-resolution electrospray ionization mass spectroscopy (HRESIMS), and NMR for structural determination as described in Example 1. As a result, the structural formula (Formula IV) was obtained. Physicochemical properties of this pigment compound are described as follows. In addition, table 2 shows the ¹³C NMR and ¹H NMR spectral data (measurement solvent: D₂O) of the pigment compound.
(1) Appearance:
Yellow powder

(2) Ultraviolet-visible absorption spectrum λₘₐₓ:
395 nm

(3) Molecular formula:

C₁₇H₂₃N₃O₄

(4) Molecular weight determined by HRESIMS:
334.1759 (M+H)⁺

**[Table 2]**

| Position | δ_{H}^{a} | δ_{C}^{b} |
|---|---|---|
| 1 | | 176.2 |
| 2 | 3.72 (1H, t, 6.2) | 55.7 |
| 3a | 2.01 (1H, m) | 31.3 |
| 3b | 1.95 (1H, m) | |
| 4a | 2.37 (2H, t, 8.1) | 24.3 |
| 4b | | |
| 5 | | 101.0 |
| 6 | 7.40 (1H, s) | 154.7 |
| 7a | 6.42 (1H, m) | 130.5 |
| 7b | | |
| 8a | 5.33 (1H, m) | 113.9 |
| 8b | | |
| 9a | 2.20 (2H, m) | 22.0 |
| 9b | | |
| 10a | 2.34 (1H, m) | 26.9 |
| 10b | 1.66 (1H, m) | |
| 11 | 3.68 (1H, ddd, 15.7, 11.7, 3.0) | 58.5 |
| 12 | 2.94 (1H, ddd, 15.8, 11.5, 5.6) | 40.8 |
| 13 | | 167.2 |
| 14 | 4.22 (1H, d, 6.3) | 58.0 |
| 15a | 2.32 (1H, m) | 26.4 |
| 15b | 2.14 (1H, m) | |
| 16a | 2.05 (1H, m) | 20.2 |
| 16b | 1.34 (1H, m) | |
| 17 | | 179.2 |

| | | |
|---|---|---|
| ^{a} Measurement at 800 MHz ^{b} Measurement at 126 MHz Measurement solvent: Heavy water | | |

### (Example 3)

The methanol aqueous solution (methanol : water = 20:80) fraction obtained in Example 1 was concentrated at 35°C under reduced pressure and subjected to high-performance liquid chromatography (ODS-80Ts; 4.6 x 250 mm; TOSOH; methanol : water = 3:17 (v/v); 0.8 ml/minute). A desired colorless pigment compound (6.9 mg) was obtained from a fraction (retention time 10 to 15 minutes).

The obtained pigment compound was subjected to single-crystal X-ray structural analysis, high-resolution electrospray ionization mass spectroscopy (HRESIMS), and NMR for structural determination as described in Example 1. As a result, the structural formula (Formula V) was obtained. Physicochemical properties of this pigment compound are described as follows. In addition, table 3 shows the ¹³C NMR and ¹H NMR spectral data (measurement solvent: D₂O) of the pigment compound.
(1) Appearance:
   Colorless powder
(2) Ultraviolet-visible absorption spectrum λₘₐₓ:
   360 nm
(3) Molecular formula:

   C₁₇H₂₅N₃O₄
(4) Molecular weight determined by HRESIMS:
   336.1915 (M+H)⁺

**[Table 3]**

| Position | δ_{H}^{a} | δ_{C}^{b} |
|---|---|---|
| 1 | | 176.1 |
| 2 | 3.70 (1H,t, 6.2) | 55.6 |
| 3a | 2.02 (1H, m) | 31.5 |
| 3b | 1.95 (1H, m) | |
| 4a | 2.45 (1H, m) | 24.0 |
| 4b | 2.29 (1H, m) | |
| 5 | | 99.3 |
| 6 | 7.38 (1H, s) | 160.6 |
| 7a | 3.66 (1H, m) | 55.2 |
| 7b | 3.20 (1H, m) | |
| 8a | 1.78 (1H, m) | 25.8 |
| 8b | 1.58 (1H, m) | |
| 9a | 1.90 (1H, m) | 23.7 |
| 9b | 1.39 (1H, m) | |
| 10a | 2.23 (1H, m) | 31.4 |
| 10b | 1.38 (1H, m) | |
| 11 | 3.22 (1H, ddd, 15.7, 11.6, 3.2) | 59.9 |
| 12 | 2.71 (1H, ddd, 15.7, 11.5, 5.2) | 41.0 |
| 13 | | 166.1 |
| 14 | 4.06 (1H, dd, 11.4, 5.4) | 58.9 |
| 15a | 2.41 (1H, m) | 27.1 |
| 15b | 1.69 (1H, m) | |
| 16a | 2.14 (1H, m) | 23.4 |
| 16b | 1.41 (1H, m) | |
| 17 | | 179.6 |

| | | |
|---|---|---|
| ^{a} Measurement at 800 MHz ^{b} Measurement at 126 MHz Measurement solvent: Heavy water | | |

### (Example 4)

The methanol aqueous solution (methanol : water = 15:85) fraction obtained in Example 1 was concentrated at 35°C under reduced pressure and subjected to high-performance liquid chromatography (ODS-80Ts; 4.6 x 250 mm; TOSOH; methanol : water = 3:22 (v/v); 0.8 ml/minute). A desired colorless pigment compound (0.6 mg) was obtained from a fraction (retention time 12 to 14 minutes).

The obtained pigment compound was subjected to single-crystal X-ray structural analysis, high-resolution electrospray ionization mass spectroscopy (HRESIMS), and NMR for structural determination as described in Example 1. As a result, the structural formula (Formula VI) was obtained. Physicochemical properties of this pigment compound are described as follows. In addition, table 4 shows the ¹³C NMR and ¹H NMR spectral data (measurement solvent: D₂O) of the pigment compound.
(1) Appearance: Colorless powder
(2) Ultraviolet-visible absorption spectrum λₘₐₓ:
   360 nm
(3) Molecular formula:

   C₂₃H₃₅N₄O₄
(4) Molecular weight determined by HRESIMS:
   465.2705 (M+H)⁺

**[Table 4]**

| Position | δ_{H}^{a} | δ_{C}^{b} |
|---|---|---|
| 1 | | 176.2 |
| 2 | 3.70 (1H, m) | 55.7 |
| 3a | 2.00 (1H, m) | 31.4 |
| 3b | 1.93(1H,m) | |
| 4a | 2.39 (1H, m) | 24.0 |
| 4b | 2.27 (1H, m) | |
| 5 | | 96.7 |
| 6 | 7.42 (1H, s) | 159.6 |
| 7a | 3.63 (1H, m) | 55.1 |
| 7b | 3.36 (1H, m) | |
| 8a | 1.77 (1H, m) | 24.0 |
| 8b | 1.69 (1H, m) | |
| 9a | 1.96 (1H, m) | 27.9 |
| 9b | 1.38 (1H, m) | |
| 10 | 1.76 (1H, m) | 41.4 |
| 11 | 3.16 (1H, dd, 12.2, 7.2) | 63.5 |
| 12 | 2.80 (1H, dt, 12.1,4.9) | 39.3 |
| 13 | | 165.3 |
| 14 | 4.11 (1H, dd, 8.8, 7.1) | 58.4 |
| 15a | 2.38 (1H, m) | 27.9 |
| 15b | 1.88 (1H, m) | |
| 16a | 2.06 (1H, m) | 27.2 |
| 16b | 1.63 (1H, m) | |
| 17 | | 180.1 |
| 18a | 1.52 (1H, m) | 33.4 |
| 18b | 1.34 (1H, m) | |
| 19a | 1.47 (1H, m) | 27.4 |
| 19b | 1.36 (1H, m) | |
| 20 | 1.38 (2H, m) | 26.0 |
| 21a | 1.87 (1H, m) | 32.1 |
| 21b | 1.83 (1H, m) | |
| 22 | 3.72 (1H, m) | 56.5 |
| 23 | | 176.7 |

| | | |
|---|---|---|
| ^{a} Measurement at 800 MHz ^{b} Measurement at 126 MHz Measurement solvent: Heavy water | | |

### (Example 5)

The methanol aqueous solution (methanol : water = 20:80) fraction obtained in Example 1 was concentrated at 35°C under reduced pressure and subjected to high-performance liquid chromatography (ODS-80Ts; 4.6 x 250mm; TOSOH; methanol : water = 3:17 (v/v); 0.8 ml/minute). A desired pigment compound taking on a yellow color (0.7 mg) was obtained from a fraction (retention time 10 to 15 minutes).

The obtained pigment compound was subjected to single-crystal X-ray structural analysis, high-resolution electrospray ionization mass spectroscopy (HRESIMS), and NMR for structural determination as described in Example 1. As a result, the structural formula (Formula VIII) was obtained. Physicochemical properties of this pigment compound are described as follows. In addition, table 5 shows the ¹³C NMR and ¹H NMR spectral data (measurement solvent: D₂O) of the pigment compound.
(1) Appearance:
   Yellow powder
(2) Ultraviolet-visible absorption spectrum λₘₐₓ:
   395 nm
(3) Molecular formula:

   C₁₉H₂₅N₃O₄
(4) Molecular weight determined by HRESIMS:
   360.1900 (M+H)⁺

**[Table 5]**

| Position | δ_{H}^{a} | δ_{C}^{b} |
|---|---|---|
| 1 | 3.64 (1H, dd, 12.6, 3.2) | 62.3 |
| 2a | 2.26 (1H, m) | 27.7 |
| 2b | 1.63 (1H, m) | |
| 3a | 2.03 (1H, m) | 24.3 |
| 3b | 1.67 (1H, m) | |
| 4a | 1.90 (1H, m) | 28.4 |
| 4b | 1.77 (1H, m) | |
| 5 | 3.74 (1H, m) | 61.9 |
| 6 | | 175.9 |
| 7 | | 100.5 |
| 8 | 7.41 (1H, m) | 154.5 |
| 9 | 6.65 (1H, m) | 130.4 |
| 10 | | 120.2 |
| 11 | 2.31 (2H, m) | 22.6 |
| 12a | 2.32 (1H, m) | 28.6 |
| 12b | 1.79 (1H, m) | |
| 13 | 3.95 (1H, m) | 53.9 |
| 14 | 2.89 (2H, m) | 35.0 |
| 15 | | 164.8 |
| 16 | 4.18 (1H,t, 4.7) | 58.5 |
| 17a | 2.14 (1H, m) | 25.2 |
| 17b | 1.92 (1H, m) | |
| 18a | 2.42 (1H, m) | 22.0 |
| 18b | 2.27 (1H, m) | |
| 19a | | 178.7 |

| | | |
|---|---|---|
| ^{a} Measurement at 800 MHz ^{b} Measurement at 126 MHz Measurement solvent: Heavy water | | |

All publications, patents, and patent applications cited herein are incorporated herein by reference in their entirety.

## Claims

1. A pigment compound of Formula I: 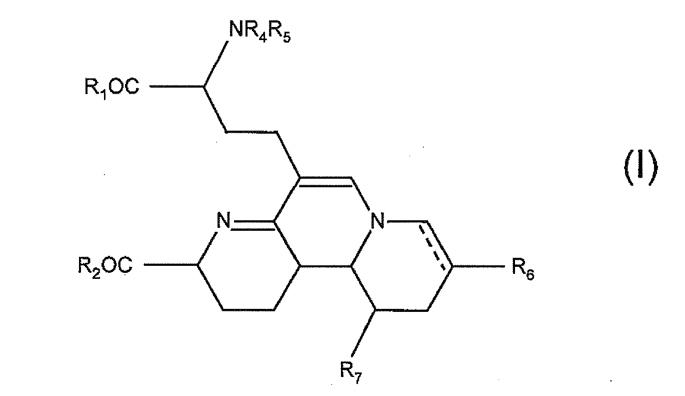 [wherein R₁ and R₂ independently represent a hydroxyl group or an alkoxy group having 1 to 4 carbon atoms, R₄ and R₅ independently represent a hydrogen atom, an acyl group having 1 to 4 carbon atoms, or an alkyl group having 1 to 4 carbon atoms, the following combination of solid and dashed lines represents a single bond or double bond, and R₆ and R₇ independently represent a hydrogen atom or a linear, branched, or cyclic alkyl group or nitrogen-containing heterocyclic group that may have an amino group and/or -COR₈ (wherein R₈ represents a hydroxyl group or an alkoxy group having 1 to 4 carbon atoms)]:

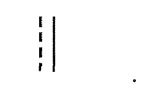

2. The pigment compound according to claim 1, wherein the compound of Formula I is a compound of Formula II: 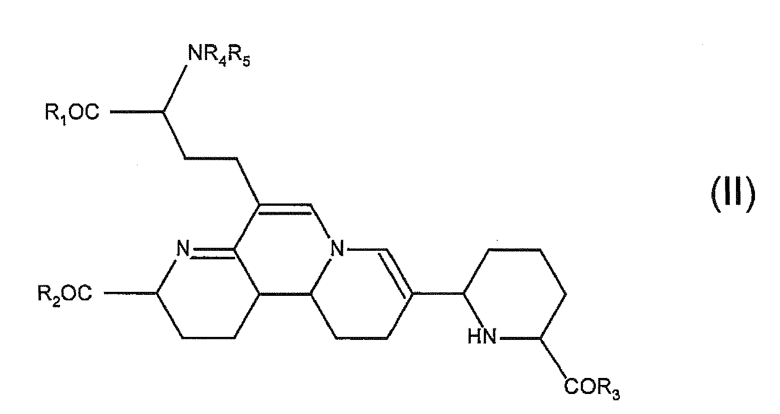 [wherein R₁ to R₃ independently represent a hydroxyl group or an alkoxy group having 1 to 4 carbon atoms, and R₄ and R₅ independently represent a hydrogen atom, an acyl group having 1 to 4 carbon atoms, or an alkyl group having 1 to 4 carbon atoms].

3. The pigment compound according to claim 2, wherein the compound of Formula II is a compound of Formula III:

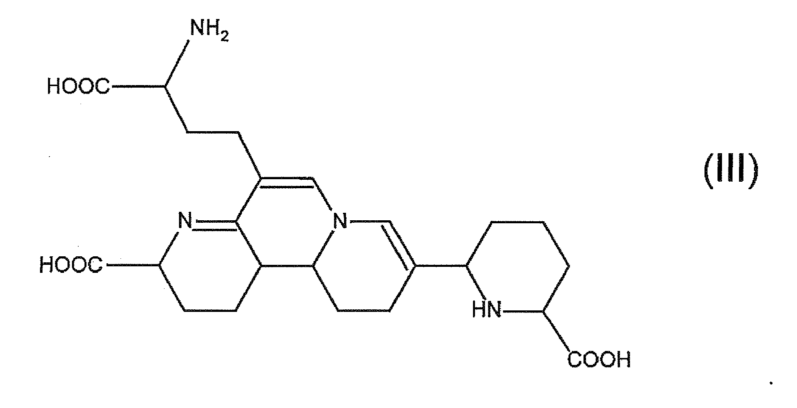

4. The pigment compound according to claim 1, wherein the compound of Formula I is a compound of Formula IV:

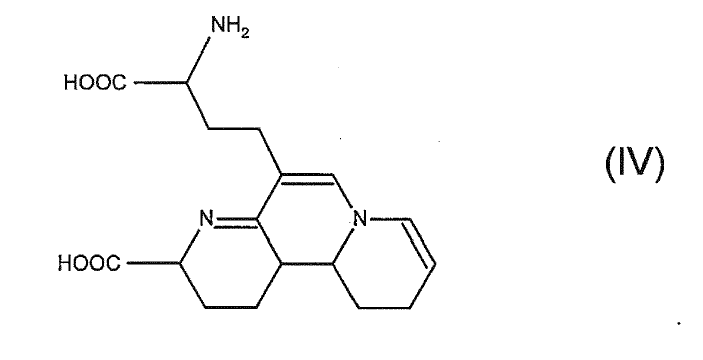

5. The pigment compound according to claim 1, wherein the compound of Formula I is a compound of Formula V:

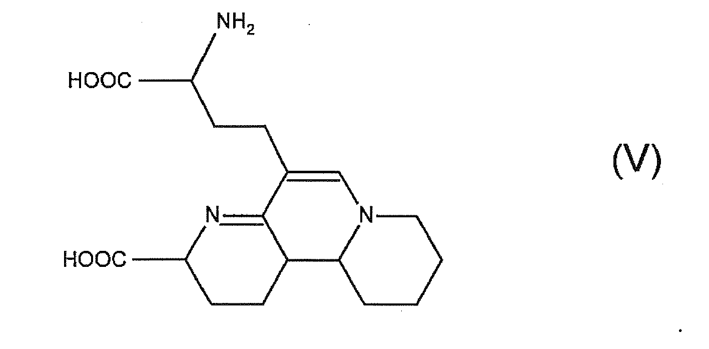

6. The pigment compound according to claim 1, wherein the compound of Formula I is a compound of Formula VI:

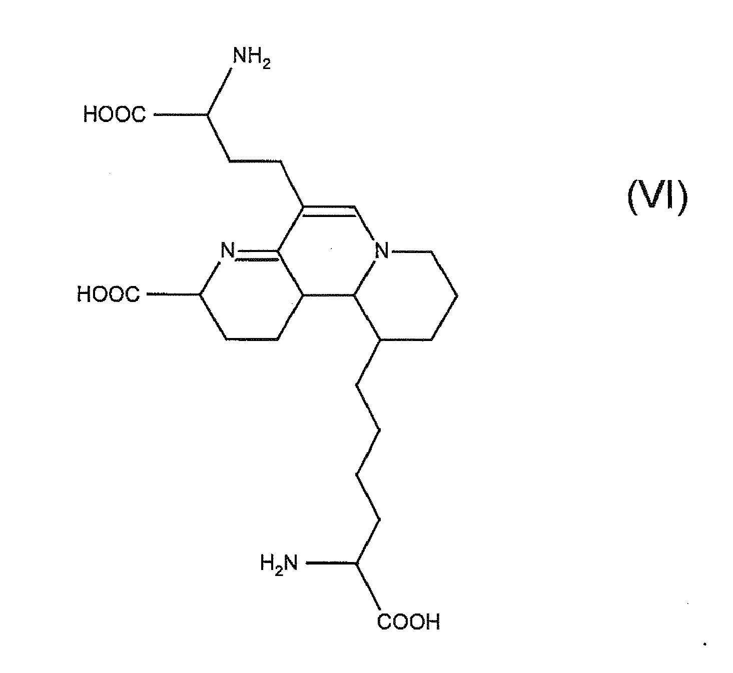

7. A pigment compound of Formula VII: 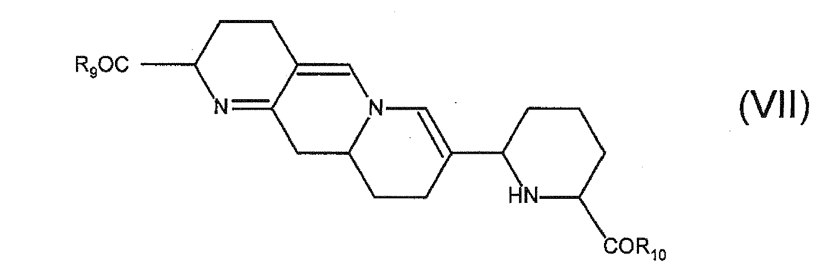 [wherein R₉ and R₁₀ independently represent a hydroxyl group or an alkoxy group having 1 to 4 carbon atoms].

8. The pigment compound according to claim 7, wherein the compound of Formula VII is a compound of Formula VIII:

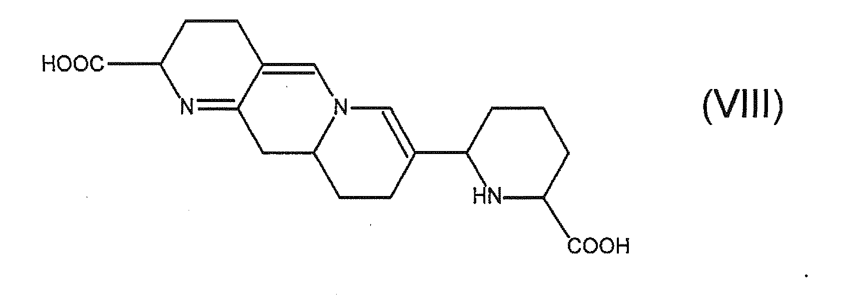

9. A method of producing the pigment compound according to any one of claims 3 to 6 or claim 8, which is a method of producing a pigment compound comprising subjecting seeds, husked rice, endosperm, or bran of the rice cultivar "Hatsuyamabuki" (FERM BP-11149) or its progeny to extraction with water or an alcohol aqueous solution and carrying out separation/purification of the pigment compound from the extract.

10. A coloring agent comprising an extractive obtained by subjecting seeds, husked rice, endosperm, or bran of the rice cultivar "Hatsuyamabuki" (FERM BP-11149) or its progeny to extraction with water or an alcohol aqueous solution and removing starch from the resultant.

11. The coloring agent according to claim 10, which comprises a fraction obtained by allowing the extractive from which starch has been removed to further adsorb to a stationary phase and then to be eluted with a solvent.

12. A coloring agent comprising the pigment compound according to any one of claims 1 to 8.
